# EUROPÄISCHE PATENTSCHRIFT

(11) **EP 0 590 201 B1**
(45) Veröffentlichungstag und Bekanntmachung des Hinweises auf die Patenterteilung: **29.07.1998**
(21) Anmeldenummer: 92116924.9
(22) Anmeldetag: 02.10.1992
(51) Int. Cl.: A61F 2/16

(54) **Vorrichtung zum Falten oder Einrollen einer gummielastischen Intraokularlinse**
Device to fold or roll an elastic intra-ocular lens
Dispositif pour plier ou enrouler une lentille intra-oculaire élastique

(43) Veröffentlichungstag der Anmeldung: 06.04.1994
(73) Patentinhaber: Klaas, Dieter Dr. med., D-86316 Friedberg (DE)
(72) Erfinder: Klaas, Dieter Dr. med., D-86316 Friedberg (DE)
(74) Vertreter: Nöth, Heinz, Dipl.-Phys.

(56) Entgegenhaltungen:
- DE-A- 4 039 119
- GB-A- 769 917
- US-A- 4 813 107
- US-A- 4 844 065

## Beschreibung

Die Erfindung betrifft eine Vorrichtung zum Falten oder Einrollen einer gummielastischen Intraokularlinse mit einem Grundkörper, zwei am Grundkörper aufeinander zu beweglich geführten Linsengreifelementen und einem zwischen den beiden Linsengreifelementen vorgesehenen Linsenaufnahmeraum, der eine am Grundkörper vorgesehene untere Auflagefläche für die zu faltende Linse aufweist.

Mit einer derartigen aus der DE 40 39 119 C1 bekannten Vorrichtung läßt sich die faltbare Linse vor dem Einsetzen in ein Implantierwerkzeug falten und somit ein erleichtertes Einsetzen des gefalteten Linsenkörpers in das Implantierwerkzeug, insbesondere zwischen den Haltespitzen der Implantierpinzette erreichen.

Aufgabe der Erfindung ist es, eine Vorrichtung der eingangs genannten Art zu schaffen, bei der der Faltvorgang auf unterschiedliche Linsentypen eingestellt werden kann.

Diese Aufgabe wird erfindungsgemäß dadurch gelöst, daß am Grundkörper ein verstellbarer Abstandhalter gelagert ist, der den Abstand der Endstellungen der beiden beim Faltvorgang aufeinander zu bewegten Linsengreifelemente bestimmt.

Bevorzugt ist der Abstandhalter in einer Vertiefung, beispielsweise in einer Führungsnut, in welcher die Linsengreifelemente an Schiebern am Grundkörper aufeinander zu beweglich geführt sind, vorgesehen. Der Abstandhalter kann in Form von Anschlägen an den Schiebern, an denen die Greifelemente befestigt sind, ausgebildet sein. Die Anschläge liegen beim Faltvorgang aneinander und sie bestimmen den Abstand der an den Schiebern befestigten Linsengreifelemente.

Es ist jedoch auch möglich, einen Abstandhalter am Grundkörper beweglich, insbesondere drehbar zu lagern, der durch seine Beweglichkeit bzw. Drehbarkeit einen verstellbaren Abstand der Endstellungen der beiden aufeinander zu bewegten Schieber bzw. Linsengreifelemente schafft. Auch dieser Abstandhalter ist in der Vertiefung bzw. Führungsnut für die beiden Linsengreifelemente vorgesehen. Er kann als Vorsprung ausgebildet sein, der in die Führungsnut ragt und er kann in seiner Höhe verstellbar sein.

Bevorzugt ist der Abstandhalter als länglicher Körper insbesondere mit rechteckigem Querschnitt ausgebildet. Wenn der Abstandhalter mit seiner Längsausdehnung parallel zur Verstellrichtung der Linsengreifelemente in der Führungsnut angeordnet ist, bildet er den maximalen Abstand der Endstellungen der beiden Linsengreifelemente. Wenn der Abstandhalter mit seiner Längsausdehnung sich quer zur Führungsnut erstreckt, bildet seine Breite den minimalen einstellbaren Abstand zwischen den aufeinander zu bewegten Linsengreifelementen. Eine stufenlose Einstellung von Abständen zwischen dem minimalen Abstand und dem maximalen Abstand ist durch entsprechendes Verdrehen des Abstandhalters in eine der Zwischenstellungen möglich.

Der Abstandhalter kann an einer Stellschraube befestigt sein. Auf diese Weise wird erreicht, daß eine stufenlose Abstandseinstellung erreicht wird und ferner, daß die Höhe mit welcher der Abstandhalter in die Führungsnut ragt ebenfalls eingestellt werden kann. Außerdem kann der Abstandhalter vollständig aus der Führungsnut entfernt werden, wenn die Stellschraube aus dem Gewinde in welchem sie im Grundkörper geführt ist herausgeschraubt wird.

Dadurch, daß die Abstandhalterfunktion in der Führungsnut, in welcher die Linsengreifelemente an Schiebern geführt sind, erreicht wird, wird der Faltvorgang durch den Abstandhalter nicht beeinträchtigt. Der Abstandhalter, d.h. beispielsweise die beiden aufeinander zu gerichteten Anschläge in den Führungsnuten an den Schiebern oder der in die Führungsnut ragende Vorsprung liegen in der Vertiefung der Führungsnut, sodaß sie nicht über die Auflagefläche auf welche die Linse vor dem Faltvorgang aufgelegt wird, überstehen. Selbst wenn die Linse durch die Linsengreifelemente nicht nur gefaltet, sondern eingerollt wird, wird der Einrollvorgang durch den Abstandhalter nicht beeinträchtigt. Durch die Abstandhalterfunktion erreicht man ein definiertes Falten oder Einrollen unterschiedlicher Linsentypen.

Für das Falten der Linse können die aufeinander zu gerichteten Anschlagflächen der Linsengreifelemente so ausgebildet sein, wie es aus der DE 40 39 119 C1 bekannt ist. Für das Einrollen von Linsen können die aufeinander zu gerichteten Anschlagflächen der beiden Linsengreifelemente jedoch auch konkav ausgebildet sein.

Anhand der Figuren wird an Ausführungsbeispielen die Erfindung noch näher erläutert. Es zeigt:
- Fig. 1: eine perspektivische Darstellung eines ersten Ausführungsbeispiels der Erfindung;
- Fig. 2: eine Seitenansicht des ersten Ausführungsbeispiels bei geöffneten Linsengreifelementen;
- Fig. 3: das erste Ausführungsbeispiel bei geschlossenen Linsengreifelementen;
- Fig. 4: ein zweites Ausführungsbeispiel in Seitenansicht;
- Fig. 5: eine Draufsicht des zweiten Ausführungsbeispiels, und
- Fig. 6: eine schnittbildliche Darstellung eines Linsengreifelements, daß bei den Ausführungsbeispielen zur Anwendung kommen kann.

Die in den Figuren dargestellten Ausführungsbeispiele einer Linsenfaltvorrichtung zum Falten einer gummielastischen Intraokularlinse 19 besitzen einen Grundkörper 4, an welchem Linsengreifelemente 1 und 2 aufeinander zu verschiebbar geführt sind. Die Linsengreifelemente 1 und 2 befinden sich an Schiebern 13 und 14, welche von Hand verschoben werden. Zur Führung der Schieber 13, 14 bzw. Linsengreifelemente 1 und 2 ist im Grundkörper 4 eine Vertiefung 3 eingeformt. Diese besteht bei den dargestellten Ausführungsbeispielen aus einer länglichen Führungsnut, insbesondere Schwalbenschwanz-Führungsnut An die Schieber 13, 14 sind Gleitstücke 15 angeformt, die der Schwalbenschwanzform der Führungsnut 3 angepaßt sind. Die Gleitstücke 15 sind in der als schwalbenschwanzförmige Führungsnut ausgebildeten Vertiefung 3 geführt.

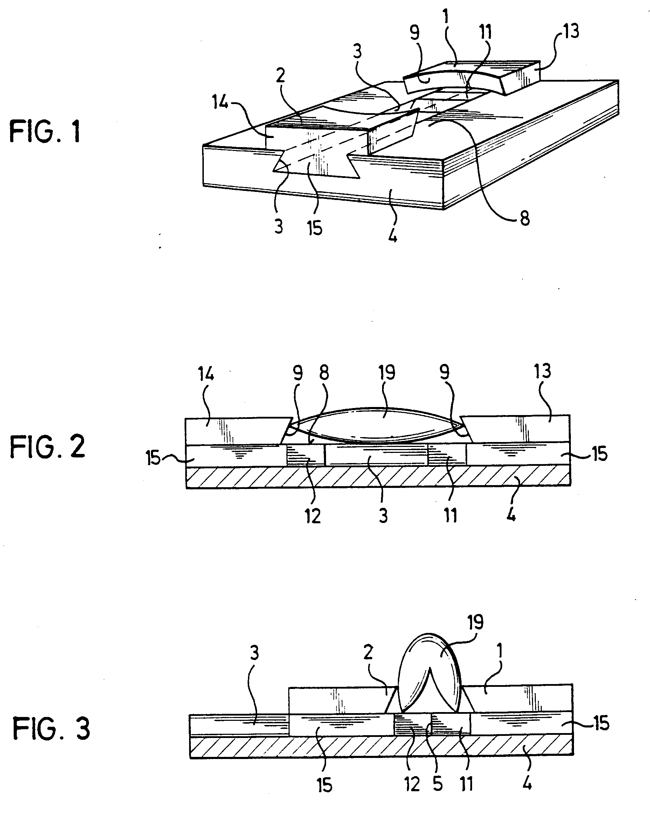

Die aufeinander zu beweglichen Linsengreifelemente 1 und 2 besitzen Anschlagflächen 9. Bei den Ausführungsbeispielen sind diese Anschlagflächen in Anpassung an die Kreisform des Linsendurchmessers leicht gewölbt, wie das aus der perspektivischen Darstellung der Fig. 1 zu ersehen ist. Ferner verlaufen sie in den Fig. 1 bis 5 im spitzen Winkel gegenüber der Oberfläche des Grundkörpers 4 bzw. einer Auflagefläche 8, auf welcher die Linse 19 bei geöffneten Linsengreifelementen (Fig. 2) flach in den Linsenaufnahmeraum zwischen den beiden geöffneten Linsengreifelementen eingelegt werden kann.

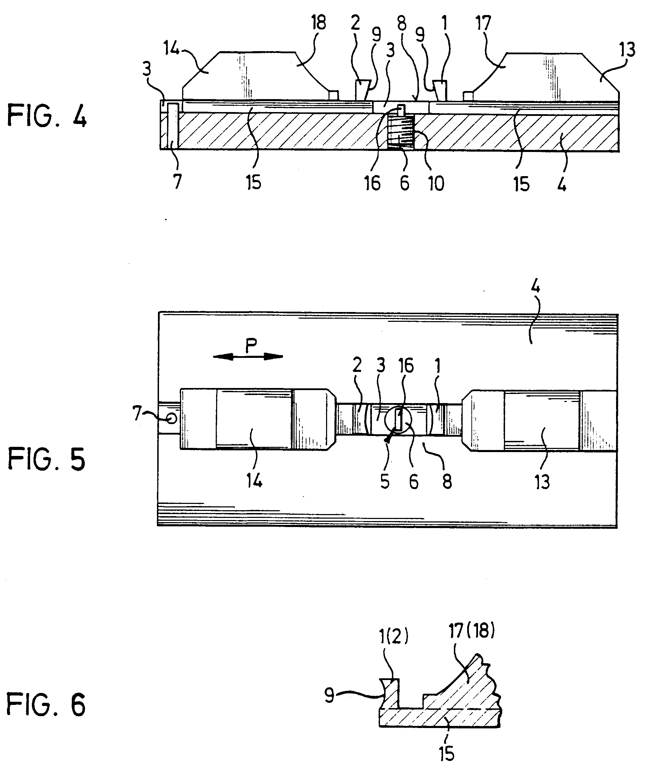

Wie die Fig. 6 zeigt, ist es jedoch auch möglich, die Anschlagfläche 9 der Linsengreifelemente konkav auszubilden, sodaß ein Einrollen der Linse möglich ist. Ein Längsschnitt eines derartigen Linsengreifelements ist in der Fig. 6 im Profil dargestellt. Auch diese Linsengreifelement in der Fig. 6 kann in Anpassung an die Kreisform des Linsendurchmessers zusätzlich gewölbt sein, wie es die Fig. 1 und auch die Draufsicht auf das zweite Ausführungsbeispiel der Fig. 5 zeigt.

Bei den dargestellten Ausführungsbeispielen ist in der Vertiefung 3 des Grundkörpers 4, welche als Führung für die Linsengreifelemente 1 und 2 am Grundkörper 4 dient, ein Abstandhalter 5 vorgesehen. Der Abstandhalter 5 gewährleistet, daß in der geschlossenen Stellung der Linsengreifelemente 1 und 2, bei welcher die Linse gefaltet wird, ein definierter Abstand zwischen den beiden Linsengreifelementen vorhanden ist. Dieser Abstand kann in Abhängigkeit von den zu faltenden Linsen eingestellt werden. Bei dem Ausführungsbeispiel der Fig. 1 bis 3 sind hierzu an den vorderen aufeinander zu gerichteten Enden der Gleitstücke 15, welche sich in der Vertiefung 3 befinden, Anschläge 11 und 12 vorgesehen, welche aufgrund ihrer Länge den Abstandhalter 5 bilden, wie es aus Fig. 3 ersichtlich ist. Durch mehrere Schieber 13, 14, welche in ihrer Länge entsprechend bemessene Anschläge 11 und 12 aufweisen, läßt sich für unterschiedliche Linsentypen der für den Faltvorgang geeignete Abstand einstellen.

Durch den Abstand 5 wird ferner gewährleistet, daß die gefaltete Linse in einer fixierten Position durch Zusammendrücken der beiden Schieber 13 und 14 positionsgerecht gehalten werden kann.

Ein weiteres Ausführungsbeispiel für einen Abstandhalter ist in den Fig. 4 und 5 dargestellt. Bei diesem Ausführungsbeispiel ist der Abstandhalter 5 in Form eines in die Vertiefung 3 ragenden Vorsprung 16 ausgebildet, der an einer Stellschraube 6 befestigt ist. Die Stellschraube 6 ist in ein Gewinde 10 der Grundkörpers 4 eingeschraubt. Durch Herausschrauben der Stellschraube 6 aus dem Gewinde 10 kann der den Abstandhalter 5 bildende Vorsprung 16 vollständig aus der als Führungsnut dienenden Vertiefung 3 entfernt werden. Es ist jedoch auch möglich, daß die Höhe mit welcher der Vorsprung 16 in die Vertiefung 3 ragt, durch die Stellschraube 6 eingestellt werden kann.

Beim dargestellten Ausführungsbeispiel besitzt der Vorsprung 16 einen rechteckigen Querschnitt. Bei der in den Fig. 4 und 5 dargestellten Stellung des Vorsprunges 16 kann der Abstand der Linsengreifelemente 1 und 2 in ihrer Schließstellung auf einen minimalen Abstand eingestellt werden. Wenn der Vorsprung 16 mit seiner Längsausdehnung parallel zur Verschiebungsrichtung (Doppelpfeil P) der beiden Schieber 13 und 14 durch Verdrehen eingestellt ist, erreicht man die Einstellung eines maximalen Abstands der Linsengreifelemente 1 und 2. Eine stufenlose Einstellung zwischen dem minimalen und dem maximalen Abstand läßt sich dadurch erreichen, daß der Vorsprung 16 in entsprechende Zwischenstellungen gedreht wird.

Bei den dargestellten Ausführungsbeispielen erreicht man ohne Beeinträchtigung des Faltvorganges bzw. Einrollvorganges eine Abstandhalterfunktion unterhalb des Linsenaufnahmeraumes bzw. unterhalb der Linsenauflagefläche 8. Die Linsengreifelemente 1 und 2 stehen über diese Fläche über und gewährleisten eine ausreichende Faltfunktion bzw. Einrollfunktion. Die beschriebene Abstandhalterfunktion wird innerhalb der Vertiefung 3, welche als Führungsnut für die beiden Schieber 13 und 14 dient, erfüllt.

Beim Ausführungsbeispiel der Fig. 4 und 5 ist noch ein ortsfester Anschlag 7 in Form eines Zylinderstiftes, welcher am Ende der Vertiefung 3 vorgesehen ist, angeordnet. Hierdurch wird erreicht, daß der Schieber 13 sich zwischen diesem Anschlag 7 und dem Abstandhalter 5 bzw. Vorsprung 16 hin und her bewegen kann. Der Schieber 13 kann ebenfalls wie der Schieber 14 vollständig aus der Vertiefung 3 entfernt werden, wenn der Vorsprung 16 beispielsweise durch Herausschrauben der Stellschraube 6 aus der Vertiefung entfernt ist. Man kann dann beispielsweise eine Reinigung der vertiefung 3 und der Schieber 13 und 14 vornehmen.

Die beiden Linsengreifelemente 1 und 2 besitzen einen bestimmten Abstand gegenüber Handgriffen 17 und 18 an den Schiebern. In diesem Abstand können Abdeckfäden, welche an dem zu faltenden Linsenkörper vorgesehen sind, eingelegt werden, sodaß der Faltvorgang oder der Einrollvorgang nicht beeinträchtigt wird.

## Patentansprüche

1. Vorrichtung zum Falten oder Einrollen einer gummielastischen Intraokularlinse (19) mit einem Grundkörper (4), zwei am Grundkörper (4) aufeinander zu beweglich geführten Linsengreifelementen (1, 2) und einem zwischen den beiden Linsengreifelementen vorgesehenen Linsenaufnahmeraum, der eine am Grundkörper vorgesehene untere Auflagefläche für die zu faltende Linse aufweist,
dadurch **gekennzeichnet,**
daß am Grundkörper (4) ein verstellbarer Abstandhalter (5) gelagert ist, der den Abstand der Endstellungen der beiden beim Faltvorgang aufeinander zu beweglichen Linsengreifelemente (1, 2,) bestimmt.

2. Vorrichtung nach Anspruch 1, dadurch gekennzeichnet, daß der Abstandhalter (5) in einer Vertiefung (3) des Grundkörpers (4) angeordnet ist, in welcher die Linsengreifelemente (1, 2) aufeinander zu beweglich geführt sind.

3. Vorrichtung nach Anspruch 1 oder 2, dadurch gekennzeichnet, daß der Abstandhalter (5) drehbar gelagert ist und durch seine Verdrehbarkeit einen verstellbaren Abstand der Endstellungen der beiden aufeinander zu bewegten Linsengreifelemente (1, 2) bildet.

4. Vorrichtung nach einem der Ansprüche 1 bis 3, dadurch gekennzeichnet, daß der Abstandhalter (5) als ein in die Vertiefung (3) ragender Vorsprung ausgebildet ist.

5. Vorrichtung nach einem der Ansprüche 1 bis 4, dadurch gekennzeichnet, daß die Höhe mit welcher der Abstandhalter (5) in die Vertiefung (3) ragt, verstellbar ist.

6. Vorrichtung nach einem der Ansprüche 1 bis 5, dadurch gekennzeichnet, daß der Abstandhalter (5) länglich ausgebildet ist und in seiner zur Verstellrichtung der Linsengreifelemente (1, 2) parallel eingestellten Längsausdehnung einen maximalen Abstand bildet und mit seiner quer zur Verstellrichtung der Linsengreifelemente (1, 2) eingestellten Längsausdehnung einen minimalen Abstand bildet.

7. Vorrichtung nach einem der Ansprüche 1 bis 6, dadurch gekennzeichnet, daß der Abstandhalter an einer Stellschraube (6) befestigt ist, die in einem Gewinde (10) im Grundkörper (4) einschraubbar ist.

8. Vorrichtung nach einem der Ansprüche 1 bis 7, dadurch gekennzeichnet, daß eines der beiden Linsengreifelemente (1, 2) zwischen einem ortsfesten Anschlag (7) und dem Abstandhalter (5) beweglich geführt ist.

9. Vorrichtung nach einem der Ansprüche 1 bis 8, dadurch gekennzeichnet, daß der Abstandhalter (5) unterhalb der Auflagefläche (8) angeordnet ist.

10. Vorrichtung nach einem der Ansprüche 1 bis 9, dadurch gekennzeichnet, daß die Linsengreifelemente (1, 2) konkave aufeinander zu gerichtete Anschlagflächen (9) aufweisen.

11. Vorrichtung nach Anspruch 1 oder 2, dadurch gekennzeichnet, daß der Abstandhalter (5) gebildet ist durch in der Vertiefung (3) geführte Anschläge (11, 12) an Schiebern (13, 14), an denen die Linsengreifelemente (1, 2) befestigt sind.

## Claims

1. A device for folding or rolling up an elastic intraocular lens (19) comprising a main body (4), two lens gripping elements (1, 2) which are guided movably towards each other on the main body (4), and a lens receiving space which is provided between the two lens gripping elements and which has a lower support surface provided on the main body for the lens to be folded, characterised in that mounted on the main body (4) is an adjustable spacer (5) which determines the spacing of the end positions of the two lens gripping elements (1, 2) which are movable towards each other in the folding operation.

2. A device according to claim 1 characterised in that the spacer (5) is arranged in a recess (3) in the main body (4), in which the lens gripping elements (1, 2) are guided movably towards each other.

3. A device according to claim 1 or claim 2 characterised in that the spacer (5) is mounted rotatably and by virtue of its rotatability forms an adjustable spacing of the end positions of the two lens gripping elements (1, 2) which are moved towards each other.

4. A device according to one of claims 1 to 3 characterised in that the spacer (5) is in the form of a projection which projects into the recess (3).

5. A device according to one of claims 1 to 4 characterised in that the height with which the spacer (5) projects into the recess (3) is adjustable.

6. A device according to one of claims 1 to 5 characterised in that the spacer (5) is of an elongate configuration and forms a maximum spacing in its longitudinal extent when set parallel to the direction of displacement of the lens gripping elements (1, 2) and forms a minimum spacing with its longitudinal extent when set transversely to the direction of displacement of the lens gripping elements (1, 2).

7. A device according to one of claims 1 to 6 characterised in that the spacer is fixed to an adjusting screw (6) which can be screwed in a screwthread (10) in the main body (4).

8. A device according to one of claims 1 to 7 characterised in that one of the two lens gripping elements (1, 2) is guided movably between a stationary abutment (7) and the spacer (5).

9. A device according to one of claims 1 to 8 characterised in that the spacer (5) is arranged beneath the support surface (8).

10. A device according to one of claims 1 to 9 characterised in that the lens gripping elements (1, 2) have concave mutually facing abutment surfaces (9).

11. A device according to claim 1 or claim 2 characterised in that the spacer (5) is formed by abutments (11, 12) guided in the recess (3) on sliders (13, 14) to which the lens gripping elements (1, 2) are secured.

## Revendications

1. Dispositif pour plier ou enrouler une lentille intra-oculaire élastique (19), comprenant un corps de base (4), deux éléments de prise de lentille (1, 2) mobiles guidés l'un vers l'autre sur le corps de base (4), et un espace de réception de lentille prévu entre les deux éléments de prise de lentille, qui présente une surface d'appui inférieure pour la lentille à plier prévue sur le corps de base, **caractérisé en ce** que sur le corps de base (4) est monté un écarteur (5) réglable qui détermine l'écartement des deux éléments de prise de lentille (1, 2) déplaçables l'un vers l'autre au cours de l'opération de pliage.

2. Dispositif selon la revendication 1, caractérisé en ce que l'écarteur (5) est disposé dans un creux (3) du corps de base (4) dans lequel les éléments de prise de lentille (1, 2) sont guidés de manière à pouvoir être déplacés l'un vers l'autre.

3. Dispositif selon la revendication 1 ou 2, caractérisé en ce que l'écarteur (5) est monté de manière tournante et forme, de par sa possibilité de rotation, un écartement des positions de fin de course des deux éléments de prise de lentille (1, 2) déplacés l'un vers l'autre.

4. Dispositif selon l'une des revendications 1 à 3, caractérisé en ce que l'écarteur (5) est réalisé sous la forme d'une saillie dépassant dans le creux (3).

5. Dispositif selon l'une des revendications 1 à 4, caractérisé en ce que la hauteur avec laquelle l'écarteur (5) dépasse dans le creux (3) est réglable.

6. Dispositif selon l'une des revendications 1 à 5, caractérisé en ce que l'écarteur (5) est allongé et forme dans son extension longitudinale réglée parallèlement à la direction de déplacement des éléments de prise de lentille (1, 2), un écartement maximum et, dans son extension longitudinale réglée transversalement à la direction de déplacement des éléments de prise de lentille (1, 2), un écartement minimum.

7. Dispositif selon l'une des revendications 1 à 6, caractérisé en ce que l'écarteur est fixé sur une vis de réglage (6) qui peut être vissée dans un filetage (10) dans le corps de base (4).

8. Dispositif selon l'une des revendications 1 à 7, caractérisé en ce que l'un des deux éléments de prise de lentille (1, 2) est guidé de manière mobile entre une butée fixe (7) et l'écarteur (5).

9. Dispositif selon l'une des revendications 1 à 8, caractérisé en ce que l'écarteur (5) est disposé en dessous de la surface d'appui (8).

10. Dispositif selon l'une des revendications 1 à 9, caractérisé en ce que les éléments de prise de lentille (1, 2) présentent des surfaces d'arrêt (9) concaves dirigées l'une vers l'autre.

11. Dispositif selon la revendication 1 ou 2, caractérisé en ce que l'écarteur (5) est constitué par des butées (11, 12) guidées dans le creux (3) et prévues sur des coulisseaux (13, 14) sur lesquels sont fixés les éléments de prise de lentille (1, 2).
